# EUROPEAN PATENT SPECIFICATION

(11) **EP 2 753 395 B1**
(45) Date of publication and mention of the grant of the patent: **29.07.2020**
(21) Application number: 12829533.4
(22) Date of filing: 07.09.2012
(51) Int. Cl.: A61M 37/00, A61N 1/30, A61N 2/00

(54) **METHOD AND DEVICE FOR DELIVERY OF SUBSTANCES**
VERFAHREN UND VORRICHTUNG ZUR VERABREICHUNG VON SUBSTANZEN
PROCÉDÉ ET DISPOSITIF POUR L'ADMINISTRATION DE SUBSTANCES

(30) Priority: 09.09.2011 AU 2011903682
(43) Date of publication of application: 16.07.2014
(73) Proprietor: International Scientific Pty Ltd, Leederville, Western Australia 6007 (AU)
(72) Inventor: EDWARDS, Jeffrey D., Nedlands, Western Australia 6009 (AU)
(74) Representative: Potter Clarkson
(86) International application number: PCT/AU2012/001067
(87) International publication number: WO 2013/033782

(56) References cited:
- WO-A1-99/39763
- WO-A1-2005/004972
- WO-A1-2005/004972
- WO-A1-2005/049135
- WO-A1-2005/049135
- US-A- 5 676 648
- US-A1- 2003 073 949
- US-A1- 2003 073 949

## Description

### Field of the Invention

The present description relates to a device for delivering an active agent to a dermal or mucosal surface comprising two releasably interconnected portions, one of which is designed to be disposed of after use.

### Background Art

The delivery of active agents to or into the skin and other biological components of the body must occur in sufficient amounts to allow the agent to achieve its purpose. However it can be difficult to achieve sufficient delivery of agents to and through various biological barriers due to difficulties in maintaining sufficient concentration in the operational environment and to the permeability barrier effect of many target biological barriers found in the integument system.

Furthermore, there is a general push, due to economic, health-related and environmental reasons, to use less of many active agents in a given composition. This provides further problems in relation to the delivery of active agents, as there may not be a sufficient concentration gradient to allow the active agent to diffuse effectively and to penetrate or partition into barriers such as the skin or mucosa.

In addition, there is a desire to provide user-friendly and convenient delivery systems that do not rely on bulky machinery or specialist techniques. It is desirable to have delivery systems that are of general applicability that can be used by a variety of individuals for a variety of needs.

There is therefore a need for devices and methods to enhance the ease of application and extent of penetration of active agents into biological barriers such as skin, using physical technologies and devices which can replace or at least compliment the previously known chemical and physical penetration enhancers.

The present description provides an improved delivery device for active agents that have, for example, a pharmaceutical, nutraceutical, biopharmaceutical, cosmeceutical, colouring, filling, plumping, anti-inflammatory, anti-aging, anti-wrinkle agents, moisturising, humectant, detergent, cleansing, bleaching, dying, fragrance, conditioning, anti-bacterial, anti-viral, antifungal, or anti-parasitic activity, wherein the user-friendly device increases the penetration of these agents into skin and other tissues of the integumentary system.

The present description relates to a delivery device for liquid, paste, flowable or free-flowing materials. Delivery devices for such materials, which may also include solids, semi-solids, gels, powdery material and material mixtures, are especially useful in the cosmetic and medical sectors. Such delivery devices should be capable of being manufactured as cost-effectively as possible on the one hand, and on the other hand be reliable and easy to use. US 5,676,648 discloses a portable iontophoresis apparatus for facilitating delivery of medication across the cutaneous membrane into adjacent underlying tissues and blood vessels. The apparatus employs a modular, detachable non-reusable medicament-containing applicator electrode which is adapted to attach to a base assembly. The apparatus is designed to be hand-held and includes a circumferential tactile electrode band on the base assembly which provides electrical connection between the skin of the user's hand and one pole of a bipolar power source housed within the base assembly. The opposing pole of the power source is connected to the applicator electrode. The user's body completes the electrical circuit between the applicator and tactile electrodes. A method for using the device for the treatment of Herpes simplex infection and related viral infections which produce similar cutaneous lesions is also disclosed. The apparatus, when used in accordance with the method described demonstrated >90% treatment efficacy in clinical trials.
WO 2005/049135 discloses an apparatus for facilitating transdermal delivery of therapeutic substances. The apparatus comprises means for producing an electromagnetic field, control means arranged to control said field producing means to alternately produce active and substantially inactive electromagnetic field portions. Each active electromagnetic field portion includes an electromagnetic field packet having a plurality of successive electromagnetic field pulses, and each substantially inactive electromagnetic field portion includes no electromagnetic field pulses. During use, when the electromagnetic field is incident on a patient, dermal permeability is increased. A corresponding method is also disclosed.

The preceding discussion of the background art was intended to facilitate an understanding of the present invention only. The discussion is not an acknowledgement or admission that any of the material referred to is or was part of the common general knowledge as at the priority date of the application.

### Description of the Invention

According to a first aspect of the present invention there is provided a device for delivering a cosmetic active agent to a dermal or mucosal surface comprising:
a) a first portion configured to define a delivery surface for presentation to a dermal or mucosal surface to deliver the cosmetic active agent; and
b) a second portion configured as a body to be held by a user further comprising a means for driving the passage of the cosmetic active agent into the dermal or mucosal surface
characterised in that: the first portion and the second portion are adapted to be releasably interconnected; and the means for driving the passage of the cosmetic active agent into the dermal or mucosal surface is housed wholly in the second portion and comprises an electromagnetophoresis dermaportation device located immediately adjacent the point of interconnection to the first portion.

According to a second aspect of the present invention there is provided a method for delivering a cosmetic active agent to a dermal or mucosal surface comprising the step of:
moving a device for delivering a cosmetic active agent to a dermal or mucosal surface over the dermal or mucosal surface
wherein the device for delivering a cosmetic active agent comprises
a first portion configured to define a delivery surface for presentation to a dermal or mucosal surface to deliver a cosmetic active agent; and
a second portion configured as a body to be held by a user
characterised in that: the first portion and the second portion are adapted to be releasably interconnected; such that the cosmetic active agent in the first portion is delivered to the dermal or mucosal surface; and the second portion further comprises a means for driving the passage of the cosmetic active agent into the dermal or mucosal surface, which means is housed wholly in the second portion, and is for driving the passage of the cosmetic active agent comprising an electromagnetophoresis dermaportation device located immediately adjacent the interconnection point of the first portion.

Those skilled in the art will appreciate that the present description is susceptible to variations and modifications other than those specifically.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs. The term "active agent" may mean one active agent, or may encompass two or more active agents.

### Preferred embodiments

### Device

In accordance with a first aspect of the present invention, there is provided a device for delivering a cosmetic active agent to a dermal or mucosal surface comprising:
a) a first portion configured to define a delivery surface for presentation to a dermal or mucosal surface to deliver the cosmetic active agent; and
b) a second portion configured as a body to be held by a user further comprising a means for driving the passage of the cosmetic active agent into the dermal or mucosal surface
characterised in that: the first portion and the second portion are adapted to be releasably interconnected; and the means for driving the passage of the cosmetic active agent into the dermal or mucosal surface is housed wholly in the second portion and comprises an electromagnetophoresis dermaportation device located immediately adjacent the point of interconnection to the first portion.

Preferably, the second portion comprises a means for driving the active agent into the dermal or mucosal surface.

### First portion

The first portion is preferably configured as a cartridge, adapted to hold the active agent.

The first portion preferably comprises a body having an outer periphery defining an inner region adapted to accommodate the active agent.

In one arrangement, the active agent may be in a solid or semi-solid form, in which case the active agent may itself define the delivery surface to allow delivery of the active agent to the dermal or mucosal surface.

In another arrangement, the first portion may comprise a cover portion adapted for transmitting the active agent, whereby the cover portion defines the delivery surface to allow delivery of the active agent to the dermal or mucosal surface. With this arrangement, the cover portion may cover the inner region and the active agent therein such that the cover portion defines the delivery surface to allow delivery of the active agent to the dermal or mucosal surface.

The active agent may be transmitted by the cover portion by passing through holes or pores in the cover portion, or by wicking of the active agent through the cover portion due to capillary action. In such a configuration, the active agent is preferably in a liquid or gel form such that the cover portion forms the delivery surface and the active agent is transmitted by the cover portion to allow delivery of the active agent to the dermal or mucosal surface.

In yet another arrangement, the inner region of the first portion may comprise a body impregnated or soaked with active agent, wherein the body defines the delivery surface to allow delivery of the active agent to the dermal or mucosal surface. The body may comprise a sponge, pad or fabric material

The first portion is preferably circular or ovoid in shape, to facilitate application of the device to the dermal or mucosal surface and enhance user comfort.

The delivery surface of the first portion that is adapted to be in contact with the dermal or mucosal surface is preferably configured in a protruding fashion. Such a configuration may be a convex in shape, or may comprise an outer face surrounded by a tapering face, wherein the outer face is the delivery surface that is presented to the dermal or mucosal surface. However, if the delivery surface comprises the active agent itself, it may be consumed during use of the device and after some use may develop a flat or concave shape.

The first portion is preferably pre-filled with the desired active agent, and may be purchased individually. Once a first portion has been applied to a dermal or mucosal surface, it may be removed from the second portion and a new first portion attached to the second portion. In this way, the first portion may be disposed of, but the second portion may be re-used.

The first portion may preferably be used for a single application of a dose of active agent before being detached and disposed of. Alternatively, the first portion may be used multiple times to deliver a single dose of active each time it is applied to a dermal or mucosal surface. For example, the first portion may comprise sufficient active agent to be used twice, three times, four times or more before being detached from the second portion and disposed of.

The outer periphery of the first portions is preferably between about 5 mm and 80 mm in diameter, more preferably between about 10 and 70 mm in diameter, most preferably about 50 mm in diameter.

The inner region of the first portions, that defines the delivery surface to allow delivery of the active agent to the dermal or mucosal surface, is preferably between about 5 mm and 60 mm in diameter, more preferably between about 10 and 50 mm in diameter, most preferably about 20 mm in diameter.

The first portions preferably have a capacity of between about 5 ³mm and 30 ³mm, most preferably about 25 ³mmm.

### Second portion

The second portion may be configured as a handle to be held by a user. Such a handle is preferably configured to be held in one hand by a user. The handle may be generally cylindrical in shape.

Alternatively, the second portion may be configured in the form of a glove to be worn by a user. The first portion is preferably releasably interconnected to the glove-shaped second portion at a location that would allow the user to apply the delivery surface of the first portion to the dermal or mucosal surface of the user, for example the first portion may be releasably interconnected to the palm of the glove-shaped second portion, or to a finger tip of the glove-shaped second portion.

In a further alternative, the second portion may be configured in the form of finger stall or finger sheath to be worn by a user. In this case, the first portion is preferably releasably interconnected to the finger stall-shaped second portion at a location that would allow the user to apply the delivery surface of the first portion to the dermal or mucosal surface of the user, for example the first portion may be releasably interconnected to the finger tip of the finger stall-shaped second portion.

If the second portion is in the form of a handle, it is preferably baton-shaped. With such an arrangement, the handle is elongate and has a longitudinal axis.

The handle is preferably substantially circular or ovoid in cross-section. The cross sectional profile is preferably between about 10 and 80 mm in diameter, more preferably about 20 mm to 50 mm in diameter.

The handle is preferably between about 80 and 200 mm in length, more preferably between about 100mm and 180 mm in length.

Preferably, the first portion is preferably releasably interconnected to an end section of the handle.

In one arrangement, the delivery surface defined by the first portion may be disposed angularly to the longitudinal axis of the handle when the first and second portions are interconnected.

In another arrangement, the delivery surface defined by the first portion may be disposed transverely to the longitudinal axis of the handle when the first and second portions are interconnected.

In yet another arrangement, the delivery surface defined by the first portion may protrude sidewardly from the handle when the first and second portions are interconnected.

### Interconnection

The first portion and the second portion are adapted to be releasably interconnected. Such a releasable interconnection may be in the form of a interference fit, snaplock connection, a threaded connection, removable adhesive connection or magnetic connection.

The first portion is adapted to be releasably interconnected to the second portion such that the first portion is applied substantially or completely externally to the second portion. The second portion may protrude into the first portion when the two portions are interconnected.

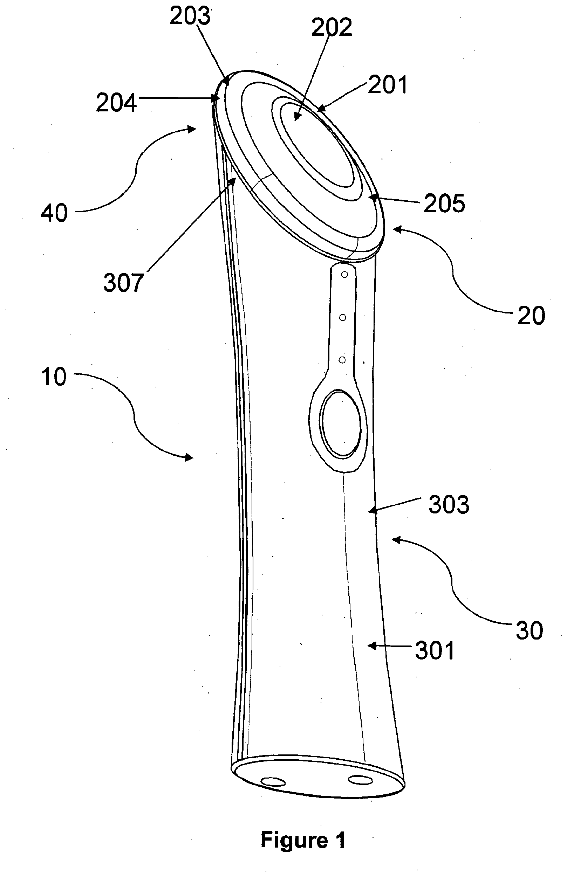

For example, the first portion may be substantially concave on the side opposite the delivery surface, such that a hollow is formed. The region of the second portion that is adapted to be releasably interconnected with the first portion may be substantially convex, in a shape mirroring that of the concave first portion surface. In this way, the second portion protrudes into the first portion when interconnected. An example of such a configuration is provided in Figure 1.

The means for driving the active agent across the dermal or mucosal surface is preferably a diffusion enhancement technique that increases the rate at which the active agent is delivered from the device for delivering an active agent to a dermal or mucosal surface, to the dermal or mucosal surface itself. The means for driving the active agent may also comprise a technique for altering the permeability of the dermal or mucosal surface to increase the amount of active agent which actually penetrates the dermal or mucosal surface.

The means for driving the active agent across the dermal or mucosal surface may be a non-powered means, such as magnetic micro- technology array, or magnetophoresis device. Alternatively, the means for driving may be a powered means, such as an electromagnetophoresis device, sonophoresis device, iontophoresis device, thermophoresis device, micro-current device or low-level laser therapy device.

For an example of a powered means for driving, the electromagnetophoresis dermaportation device of PCT/AU2004/001599 may be used to enhance penetration of the active agents into the skin. Other examples of powered devices include US20020147424.

Preferably, the powered means for driving the active agent into the dermal or mucosal surface is an electromagnetophoresis dermaportation device comprising:
an electromagnetic field generating device including a coil which has electrical connectivity at both ends to allow unidirectional flow of current;
a control device arranged to control said field generating device to alternately produce active and substantially inactive electromagnetic field portions, each said active electromagnetic field portion having a frequency of between 10Hz and 100Hz at a field strength of between 1 and 50 Gauss
   and comprising a plurality of generally rectangular electromagnetic field pulses wherein each electromagnetic field pulse has a duration of between 25µs and 100ms, each said substantially inactive electromagnetic field portion including no electromagnetic field pulses, wherein the duration of the inactive electromagnetic field portion is longer than the duration of the active electromagnetic field portion;
   wherein during use when the electromagnetic field is incident on the dermal or mucosal surface, permeability is increased

The electromagnetic field generating device preferably comprises a solid state switching device. The solid state switching device may comprise a transistor such as a bipolar transistor connected in series with the coil.

Preferably, the control device is arranged to produce an energisation signal useable to control switching of the solid state switching device to produce active and substantially inactive energisation signal portions, each active energisation signal portion including a plurality of energisation signal pulses and a substantially inactive energisation signal portion including no energisation signal pulses, and wherein the active energisation signal portion produces the active electromagnetic field portion and the inactive energisation signal portion produces the inactive electromagnetic field portion.

The control device further preferably comprises a microcontroller, which may be programmable by a user so that an electromagnetic signal corresponding to a predetermined therapeutic substance delivery plan is produced. The microcontroller may be programmed such that dermal permeability is increased at one or more specific times, permeability is increased for a specific period of time, and so on. For example, it may be configured so that the apparatus carries out a specific treatment plan, for example by generating an appropriate energisation signal pattern and using the energisation signal to apply one or more specific electromagnetic field patterns to a target area of a patient at specific times, for a specific time duration and/or in response to the specific characteristics of the active agent being delivered.

The electromagnetophoresis dermaportation device preferably produces and delivers an electromagnetic field to the dermal or mucosal surface wherein the field delivered is defined by a mnemonic profile of:
[(A-B-C-D)E], [(A₁-B₁ -C₁-D₁)E₁]
where,
A and A₁ respectively define the number of 400 s time units that the electromagnetic field pulse is on for wherein each of A and Ai is a number between 0.1 and 10; B and B₁ respectively define is the number of 400 s time units the field is off for wherein B is a number between 0.1 and 100, while B₁ is a number between 0.1 and 100; C is a number between 1 and 255, which defines the number of times the A and B combination is repeated, while C₁ is also a number between 1 and 255 and defines the number of times the A₁ and B₁ combination is repeated; D and D₁ respectively define the number of 400µs time units that the field is off for wherein D is a number between 0 and 255, while D₁ is a number between 0 and 255; E defines the number of times the A to D envelope is executed before moving onto the [(A₁-B₁-C₁-D₁)E₁] packet, while E₁ defines the number of times the A₁ to D₁ envelope is executed before moving onto the next mnemonic profile wherein E and E₁ are respectively numbers between 1 and 25; and
and wherein during use when the electromagnetic field is incident on the dermal or mucosal surface, the cellular environment into the surface is modified.

According to the present description, the mnemonic profile selected will differ according the drug administered. The following table illustrates various field parameters for a range of different drugs.

| **Field parameters** | **Compound** |
|---|---|
| [(1,1,12,255)5], [(1,35,72;0)2] | Diclofenac diethylammonium salt |
| [(1,1,12,255)5], [(1,35,72,0)2] | Hydrocortisone in PBS |
| [(1,1,12,255)5], [(1,35,72,0)2] | GLANVAC vaccine |
| [(1,1,24,255)5], [(1,35,72,0)2] | Tetracaine HCL |
| [(1,1,12,255)5], [(1,35,72,0)2] | Tetracaine (4%) |
| [(1,1,12,255)5], [(1,35,72,0)2] | Ametop (tetracaine gel) |
| [(1,1,12,255)5], [(1,35,72,0)2] | 5-aminolevulinic acid |
| [(1,1,12,255)5], [(1,35,72,0)2] | Naltrexone in PBS |
| [(1.1.12.255)5]. [(1,35,72,0)2] | 5mg/ml Lidocaine HCL in PBS |
| [(1,1,12,255)5], [(1,35,72,0)2] | 5mg/ml Lidocaine HCL in PBS |
| [(1,1,12,255)5], [(1,35,72,0)2] | Lidocaine in PBS |
| [(1,1,12,255)5], [(1,35,72,0)2] | Lidocaine HCL, prilocaine HCL combination,, PBS |
| [(1,1,12,255)5], [(1,35,72,0)2] | Lidocaine in PBS |
| [(1,1,255,0)1], [(1,1,255,0)1] | |
| [(1,1,12,255)5], [(1,35,72,0)2] | 5mg/ml Lidocaine HCL in PBS |
| [(1,1,12,255)5], [1,35,72,0)1] | |
| [(1,1,12,255)5], [(1,35,72,0)2] | 5mg/ml Lidocaine HCL in PBS |
| [(1,1,12,255)10], [(1,35,72,0)2] | |
| [(1,1,12,255)5], [(1,35,72,0)2] | 5mg/ml Lidocaine HCL in PBS |
| [(1,1,24,255)5], [(1,35,72,0)2] | |
| [(1,1,12,255)5], [(1,35,72,0)2] | Lidocaine HCL |
| [(1,1,24,255)5], [(1,35,72,0)2] | |
| [(1,1,24,255)5], [(1,35,72,0)2] | Lidocaine Hcl 5mg/ml |
| [(1,1,12,255)5], [(1,35,72,0)2] | Lidocaine HCL 5mg/ml |
| [(1,1,2,255)5], [(1,1,2,255)5] | |
| [(1,1,12,255)5], [(1,35,72,0)2] | Lidocaine HCL 5mg/ml |
| [(2.5,255,3),255)]10],[(2.5,255,3),255)10] | |
| [(1,1,12,255)5], [(1,35,72,0)2] | Lidocaine HCL 5mg/ml |
| [(2.5,255,3),255)10],[(2.5,255,3),255)10] | |
| [(1,1,12,255)5], [(1,35,72,0)2] | Lidocaine HCL 5mg/ml |
| [(2.5,2.5,2)25000)5], [(-2.5,-2.5,2)25000)1] | |
| [(1,1,12,255)5], [(1, 35, 72,0)2] | Lidocaine HCL 5mg/ml |
| [(2.5,2.5,2)25000)5], [(-2.5,-2.5,2)25000)1] | |

Alternatively, a non-powered driving device comprising a magnetic array comprising one or more pairs of displaced dipolar magnetic elements linked by a magnetic return wherein the magnetic return is orientated on the surfaces of the dipole pair distal to the dermal or mucosal surface may be used. Use of such a non-powered device in the present device for delivering an active agent to a dermal or mucosal surface may be enhanced by reciprocal, rotational or orbital movement of the delivery device over the dermal or mucosal surface, which imparts a movement to the magnetic device so that the dermal or mucosal surface in contact with the active agents in the first portion will be subject to alternating polarities of magnetic field and alternating magnetic gradients in response to said reciprocal, rotational or orbital movement.

The means for driving is preferably located in the second portion immediately adjacent the point of interconnection to the first portion.

### Separate portions

In accordance with the present description, there is provided the first portion of the device for delivering an active agent to a dermal or mucosal surface, configured to define a delivery surface for presentation to a dermal or mucosal surface to deliver an active agent, in isolation from the second portion of the delivery device.

Separately, there is provided the second portion of the device for delivering an active agent to a dermal or mucosal surface, configured as a body to be held by a user further comprising a means for driving the passage of the active agent into the dermal or mucosal surface, in isolation from the first portion of the delivery device. Preferably the second portion further comprises a powered means for driving the active agent into the dermal or mucosal surface, most preferably a electromagnetophoresis dermaportation device.

Generally, it is anticipated that the first portions of the delivery device will be available to users separately to the second portion. The first portion will generally be of a disposable nature, and multiple packs of first portions will be purchased by a user. The second portion will generally be retained by the user for use multiple times.

The first portions will be releasably interconnected to the second portion, the active agent applied to the dermal or mucosal surface, then the first portion released from the second portion and disposed of. Different first portions comprising different active agents can be used with one single second portion, by removing one first portion comprising a first active agent, and replacing it with a second first portion comprising a second active agent.

### Active agents

Suitable active agent(s) that can be delivered include any active agent(s) exhibiting negative magnetic susceptibility and any active agent(s) having therapeutic, cosmetic, restorative, antimicrobial, anti-fungal, cleaning or disinfectant beneficial properties when administered to a surface as described herein. When the device according to the present description is employed to assist in the passage of active agents across a biological barrier such as such as skin the class of active agents, include, for example, proteins, peptides, nucleotides, anti-obesity drugs, corticosteroids, analgesics, anti-fungal agents, oncology therapies, cardiovascular agents, anti-inflammatory agents, non-steroidal anti-inflammatory agents, anti-arrhythmic agents, antibiotics, anticoagulants, antidepressants, antidiabetic agents, antiepileptics, antihistamines, anti-hypertension agents, antineoplastic agents, immunosuppressants, anti-thyroid agents, antiviral agents, sedatives, astringents, beta-adrenoceptor blocking agents, diuretics, muscle reactants, prostaglandins, sex hormones, anti-allergic agents, stimulants, vasodilators, xanthenes, antioxidants, vitamins, nutrients, skin restorative agents, hair care or restorative agents and those active agents delivered as nutraceuticals, cosmeceutical or cosmetics to or through a biological barrier such as skin.

More specific groups of active agents that may be delivered by the device of the present description include: colouring agents including coloured cosmetics, concealing agents, skin whitening agents, tanning agents; cleaning agents including exfoliants, dermabrasives, anti-bacterials, anti-fungals, sloughing agents, pore treatment agents, oils, creams, gels and serums; moisturising agents, rehydrating agents and skin nourish agents; skin treatment agents for inflammation, redness, pimples and bacterial infections; antiperspirants, deodorants, and perfumes; fillers, plumping agents and agents that alter the skin's topography; anti-inflammatory agents, analgesic agents and anaesthetic agents

Non-limiting examples of pharmaceutical and biopharmaceutical active agents which could be delivered to the skin using the device of the present description include:
a) steroids such as sulfonamides, triamcinolone, betamethasone, budesonide, cortisone, dexamethasone, hydrocortisone, methylprednisolone, prednisolone, prednisone sodium phosphate, fluorometholone, rimexolone, medrysone alcohol, 11 -desoxcortisol, and anecortave acetate and the like
b) anti-inflammatory agents such as hormonal agents, clobetasol, dexamethasone, acetyl salicylic acid, glycyrrhizic acid or glycyrrhetic acid, hydrocortisone, prednisolone, prednisone, non-hormonal agents, allopurinol, aspirin, indomethacin, phenylbutazone and the like;
c) antibiotics such as the cephalosporins, chloranphenical, gentamicin, Kanamycin A, Kanamycin B, the penicillins, ampicillin, streptomycin A, antimycin A, chloropamtheniol, metromidazole, oxytetracycline penicillin G, the tetacyclines and the like;
d) anti-acne agents, such as salicylic acid and benzoyl peroxide;
e) antimicrobial or antifungal agents such as, for example, caprylyl glycol, triclosan, phenoxyethanol, erythromycin, tolnaftate, nystatin or clortrimazole;
f) chelating agents, such as EDTA and the like;
g) topical analgesics, such as benzocaine, tetracaine, lidocaine or procaine and the like;
h) peptides with either therapeutic benefit or cosmetic benefit comprising between two and 20 amino acid residues, preferably, between three and 10 amino acid residues (cosmetic peptides such as palmitoyl pentapeptide or argireline which have beneficial effect on skin cells eg whitening, free-radical scavenging, anti-aging, stimulation of collagen synthesis, moisturizing, antimicrobial, anti-inflammatory, or anti-irritant) and the like.

Non-limiting examples of neutraceutical active agents which could be delivered to the skin using the device of the present description include:
a) Vitamins and nutrients including essential amino acids and the like;
b) Electrolyte replacements such as potassium chloride and the like;
c) Antioxidants or free-radical scavengers: such as ascorbic acid, its fatty esters and phosphates, tocopherol and its derivatives, N-acetyl cysteine, sorbic acid and lipoic acid and the like;

Non-limiting examples of cosmetic active agents which could be delivered to the skin using the device of the present description include:
a) Moisturising agents and emollients: occlusive agents e.g. hydrocarbons such as petrolatum, silicone containing agents such as dimethicone, cyclomethicone, fatty acids and alcohols such as lanolin acid or alcohol, sterols such as cholesterol, waxes and fats such as cocoa butter, carnuba wax and bees wax; humectants e.g. glycosamines such as hyaluronic acid, glycerine, honey, urea, lactic acid, α-hydroxy acids, propylene glycol etc;
b) Anti-aging compounds: such as retinoids or hydroxy acids;
c) Deodorants: including alcohol based deodorants; deodorants containing antimicrobial agents such as triclosan or metal chelating agents; perfumes and essential oils;
d) Antiperspirants: including aluminium based compounds such as aluminium chloride, aluminium chlorohydrate, and aluminium-zirconium compounds (aluminium zirconium tetrachlorohydrex gly and aluminium zirconium trichlorohydrex gly); potassium alum and ammonium alum;
e) Fragrances: such as essential oils, musk, alcohols (eg furaneol, menthol), esters (eg fructone, ethyl methylphenylglycidate), ketones (eg dihydrojasmone), lactones (eg coconut odour, jasmine lactone);
f) Astringents: such as clove oil, menthol, camphor, eucalyptus oil, eugenol, menthyl lactate, witch hazel distillate, aluminium based compounds and the like;
g) Skin lightening agents: such as liquorice, ascorbyl phosphates, hydroquinone or kojic acid and the like;
h) Sun protecting agents (organic or inorganic): such as avobenzone, oxybenzone, octylmethoxycinnamate, titanium dioxide or zinc oxide;
i) Exfoliating agents (chemical or physical): such as N-acetyl glucosamine, mannose phosphate, hydroxy acids, lactobionic acid, peach kernels, or sea salts and the like;
j) Self-tanning agents: such as dihydroxyacetone;
k) Colouring agents for skin and mucosal surfaces;
l) Plumping agents and fillers: such as hyaluronic acid or hyaluronate.
m) Other agents such as aloe vera.

Additional agents that could be delivered to the skin surfaces include additional nutritional type ingredients, such as vitamins, minerals, amino acids, vitamin E, and folic acid; sensate ingredients, such as those providing cooling (such as menthol), tingle, or heat sensations (such as capsaicin or capsicum oil); colorants or other aesthetic agents; and combinations thereof. Essential oils may also be delivered by the device according to the present description, such as oils of lavender, rose, rosemary, spearmint, peppermint, wintergreen, eucalyptus, lemon, lime, grapefruit, and orange.

The above list of active agent(s) may be applied in a controlled manner, using the method of the present description. This list is not exhaustive. Preferably, any active agent(s) that can be delivered systemically or topically can potentially be delivered using the present teaching.

The active agent may be in the form of a solid, gel, paste, liquid, thermo-reversible gel or paste, etc.

While the active agent(s) may be provided and used alone with the device, in many situations the active agent will be included in a formulation either alone or in combination with one or more other active agents. Where the formulation is to provide a pharmaceutical and/or biopharmaceutical benefit, the number of active agent(s) included in the formulation may preferentially be quite selective. Where the formulation provides a nutraceuticals, cosmetic and/or cosmeceutical effect, the number of active agents may be much greater in number.

The formulation employed in the delivery process may include additives such as other buffers, diluents, carriers, adjuvants or excipients. Any pharmacologically acceptable buffer that is magnetically inert or neutral or which has a magnetic susceptibility that is either paramagnetic in nature or greater than that of the active agent(s) being delivered, may be used, e.g., tris or phosphate buffers. Other agents may be employed in the formulation for a variety of purposes. For example, buffering agents, preservatives, co-solvents, surfactants, oils, humectants, emollients, chelating agents, stabilizers or antioxidants may be employed. Water soluble preservatives which may be employed include, but are not limited to, benzalkonium chloride, chlorobutanol, thimerosal, sodium bisulfate, phenylmercuric acetate, phenylmercuric nitrate, ethyl alcohol, methylparaben, polyvinyl alcohol, benzyl alcohol and phenylethyl alcohol. A surfactant may be Tween 80. Other vehicles that may be used include, but are not limited to, polyvinyl alcohol, povidone, hydroxypropyl methyl cellulose, poloxamers, carboxymethyl cellulose, hydroxyethyl cellulose, purified water, etc. Tonicity adjustors may be included, for example, sodium chloride, potassium chloride, mannitol, glycerin, etc. Antioxidants include, but are not limited to, sodium metabisulfite, sodium thiosulfate, acetylcysteine, butylated hydroxyanisole, butylated hydroxytoluene, etc.

The indications, effective doses, contra-indications, vendors etc, of the active agents in the formulations are available or are known to one skilled in the art.

The active agents may be present in individual amounts of from about 0.001 to about 5% by weight and preferably about 0.01% to about 2% by weight. However, it is contemplated that the active agents may be present in individual amounts greater than this, for example up to 100%.

Suitable water soluble buffering agents that may be employed include sodium carbonate, sodium borate, sodium phosphate, sodium acetate, sodium bicarbonate, etc., as approved by the US FDA for the desired route of administration. These agents may be present in amounts sufficient to maintain a pH of the system of between about 2 to about 9, preferably about 4 to about 8, more preferably 4.5, 5, 5.5, 6, 6.5, 7 or 7.5 (or any pH in between). As such the buffering agent may be as much as about 5% on a weight to weight basis of the total formulation. Electrolytes such as, but not limited to, sodium chloride and potassium chloride may also be included in the formulation where appropriate.

The active agents to be delivered using the device of the present description may be provided in a matrix layer. If the active agent delivered by the device of the present description is contained within a matrix, the matrix preferably allows the active agent to diffuse or exit the matrix in some manner and contact the dermal or mucosal barrier, perhaps by being transmitted through a cover portion of the first portion to the dermal or mucosal barrier.

The matrix is preferentially prepared from a polymer or copolymer prepared from e.g., polyisobutylene, ester of polyvinyl alcohol, polyacrylic and polymethacrylic acid esters, natural rubber, polymers of styrene, isoprene, and styrene-butadiene or silicone polymers, resin components, such as, saturated and unsaturated hydrocarbon resins, derivatives of abietyl alcohol and of beta-pinene, plasticizers, such as phthalic acid esters, triglycerides and fatty acids, as well as a series of other substances known to those skilled in the art.

Matrix biocompatible polymers that might be used include compounds such as polycaprolactone, polyglycolic acid, polylactic acid, polyanhydrides, polylactide-co-glycolides, polyamino acids, polyethylene oxide, acrylic terminated polyethylene oxide, polyamides, polyethylenes, polyacrylonitriles, polyphosphazenes, poly(ortho esters), sucrose acetate isobutyrate (SAIB), and other polymers such as those disclosed in U.S. Patent Nos. 6,667,371; 6,613,355; 6,596,296; 6,413,536; 5,968,543; 4,079,038; 4,093,709; 4,131,648; 4,138,344; 4,180,646; 4,304,767; 4,946,931.

The matrix containing the active agents may also be prepared from thermosetting polymers such as tetra-substituted ethylene diamine block copolymers of ethylene oxide and propylene oxide (e.g., poloxamine); polycarbophil; and polysaccharides such as gellan, carrageenan (e.g., kappa-carrageenan and iota-carrageenan), chitosan and alginate gums.

The matrix may also be a hydrogel, being a gel prepared with hydrophilic polymers, and these materials are well known in the art, frequently being used as part of biomedical electrodes, such as are described in U.S. Pat. Nos. 6,631,294 and 6,845,272.

Examples of hydrophilic polymers useful for the preparation of hydrogels are polyacrylate, polymethacrylate, polyacrylamide, poly(vinyl alcohol), poly(ethylene oxide), poly(ethylene imine), carboxy-methylcellulose, methylcellulose, poly(acrylamide sulphonic acid), polyacrylonitrile, poly(vinylpyrrolidone), agar, dextran, dextrin, carrageenan, xanthan, and guar. The preferred hydrogels are acrylates and may be, for example, preferably made from acrylic esters of quaternary chlorides and/or sulfates or acrylic amides of quaternary chlorides; polymers of this type are disclosed in U.S. Pat. No. 5,800,685. The hydrophilic polymers will generally constitute from about 1 to about 70%, preferably about 5 to about 60%, more preferably about 10 to about 50%, by weight of the hydrogel.

In a highly preferred form of the present teaching, a topical formulation for delivery to a subject is prepared by selecting a desired amount of active agent. The agent is then preferably placed in a suitable delivery matrix. The amount of the active agent to be administered and the concentration of the compound in the topical formulation depends upon the diluent, delivery system or device selected, the clinical or cosmetic condition of the subject, the side effects and the stability of the active agent in the matrix.

### Method

The present invention further provides a method for delivering an active agent to a dermal or mucosal surface comprising the steps of:
moving a device for delivering an active agent to a dermal or mucosal surface over the dermal or mucosal surface wherein the device for delivery comprises a first portion configured to define a delivery surface for presentation to a dermal or mucosal surface to deliver an active agent; and a second portion configured as a body to be held by a user characterised in that the first portion and the second portion are adapted to be releasably interconnected, such that the cosmetic active agent in the first portion is delivered to the dermal or mucosal surface; and
the second portion further comprises a means for driving the passage of the cosmetic active agent into the dermal or mucosal surface, which means is housed wholly in the second portion, and is for driving the passage of the cosmetic active agent comprising an electromagnetophoresis dermaportation device located immediately adjacent the interconnection point of the first portion.

### Non-limiting illustration of the invention

Further features of the present teaching are more fully described in the following description of several non-limiting embodiments thereof.
Figure 1 is perspective view of a delivery device according to a first embodiment, with first and second portions thereof shown in an interconnected condition;
Figure 2 is a view similar to Figure 1, except that the first and second portions are shown in a detached condition; Figure 3 is affront view of the delivery device in the condition shown in Figure 1 ;
Figure 3 is affront view of the delivery device in the condition shown in Figure 1;
Figure 4 is a perspective view of the first portion illustrating in particular the delivery surface thereof;
Figure 5 is a further perspective view of the first portion illustrating the delivery surface thereof;
Figure 6 is a front view of the first portion;
Figure 7 is a side view of the first portion;
Figure 8 is further perspective view of a delivery device according to the first embodiment, with first and second portions thereof shown in an interconnected condition;
Figure 9 is perspective view of a delivery device according to a second embodiment, with first and second portions thereof shown in an interconnected condition;
Figure 10 is perspective view of a delivery device according to a third embodiment, with first and second portions thereof shown in an interconnected condition; and
Figure 11 is perspective view of a delivery device according to a fourth embodiment, with first and second portions thereof shown in an interconnected condition.

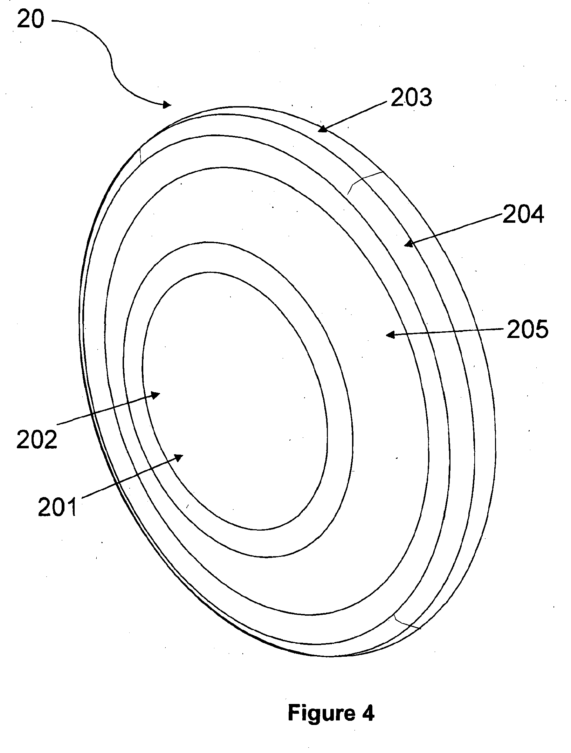

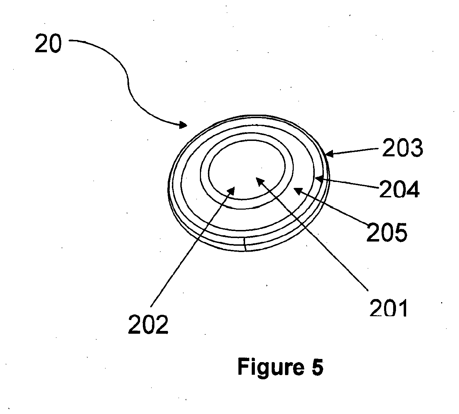

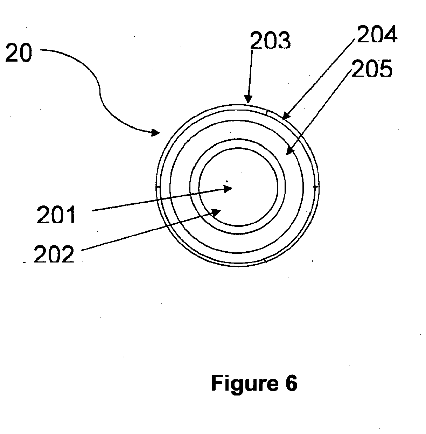

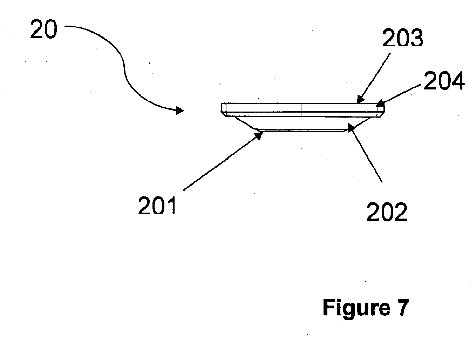

Referring to Figures 1 to 8, the first embodiment is directed to a device 10 for delivering an active agent to a dermal or mucosal surface comprising a first portion 20 configured to define a delivery surface 201 for presentation to a dermal or mucosal surface to deliver an active agent 202 and a second portion 30 configured as a handle.

In this embodiment, the first portion 20 is configured as a cartridge adapted to hold the active agent 202, with a body 203 having an outer periphery 204 defining an inner region 205 adapted to accommodate the active agent 202. The first portion 20 is circular to enhance user comfort. The delivery surface 201 is convex in shape.

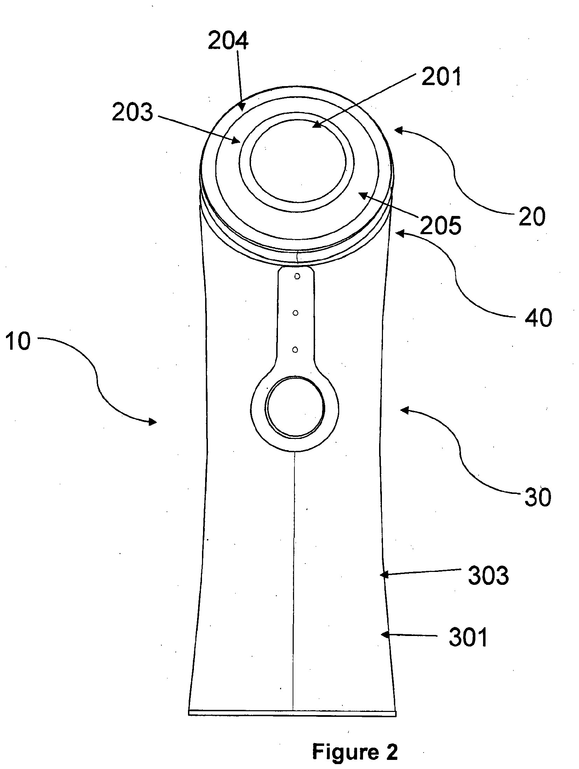

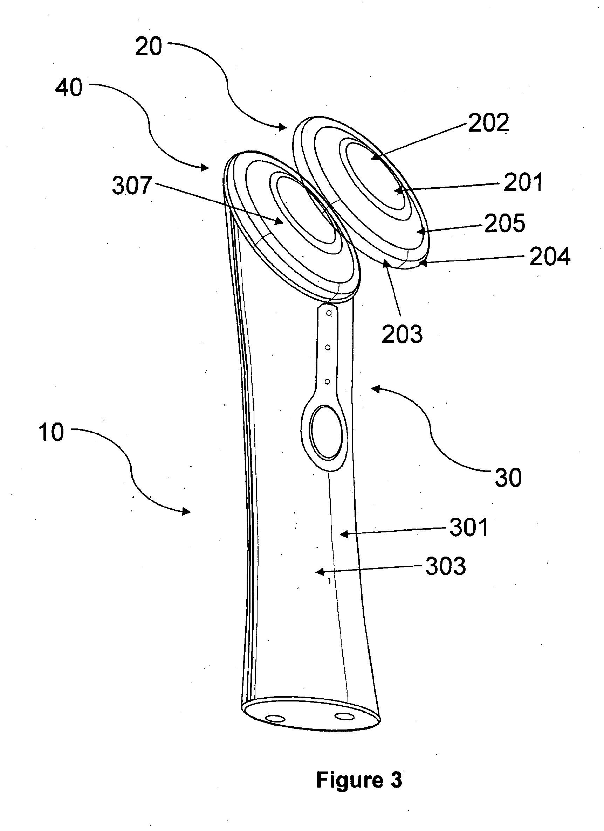

The first portion 20 can be interconnected with the second portion 30 via a releasable interference fit connection. In Figure 3, the two portions are not interconnected, however, the first portion is shown interconnected with the second portion in Figures 1 and 2.

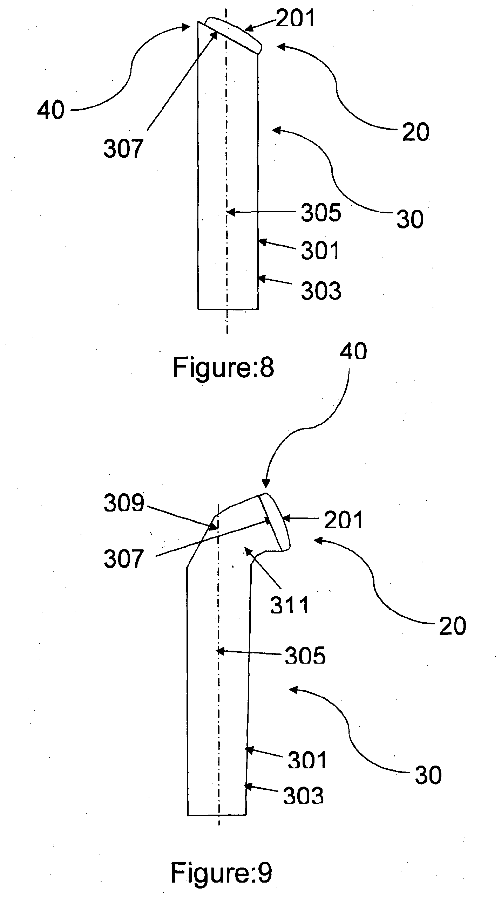

In the first embodiment, the second portion 30 comprises an elongate body 301 configured to define a handle 303 adapted to be held in one hand by a user. The elongate body 301 defines a longitudinal axis 305 as shown in Figure 8.

The elongate body 301 has an end section 307 to which the first portion 20 is adapted to be detachably connected,

In the arrangement illustrated, the end section 307 of the second portion 20 is of wedge configuration, whereby the delivery surface 201 defined by the first portion 20 is disposed angularly to the longitudinal axis 305 as illustrated in Figure 8. The angular disposition of the delivery surface 201 is advantageous as it provides an ergonomic disposition with respect to the handle 303 for presentation to the dermal or mucosal surface.

The handle 303 is generally circular or otherwise rounded in cross-section to offer a comfortable grip when being held in one hand by a user.

The end section 307 is configured to accommodate the cartridge defined by the first portion 20. The end section 307 may incorporate a recess (not shown) into which a mating section of the cartridge can be received to align and positively located the first and second portions as they are moved into an interconnected condition. The positive location may also assist in maintaining the interconnected condition between the first and second portions.

In this embodiment, the first and second portions are releasably interconnected by way of an interference fit, although other connection arrangements are possible as explained earlier.

The means for driving the passage of the active agent 40 across the dermal or mucosal surface is located inside the second portion 30, immediately adjacent the end 301 of the second portion 30 that interconnects with the first portion 20. The second portion 30 optionally further comprises batteries to provide electricity to run the means for driving, if that means is a powered means such as an electromagneto-phoresis device. Alternatively, the second portion 30 may have a connection, such as an electrical plug and wires, which can connect the device for delivering an active agent to the AC power grid to provide electricity to run the driving means.

Referring now to Figure 9, there is shown a delivery device according to a second embodiment. The delivery device according to the second embodiment is similar in many respected to the first embodiment and corresponding reference numerals are used to identify similar parts. In this second embodiment, the end section 307 is configured as an extension 309 incorporating an internal bend 311 to provide for the angular disposition of the delivery surface 201 defined by the first portion 20.

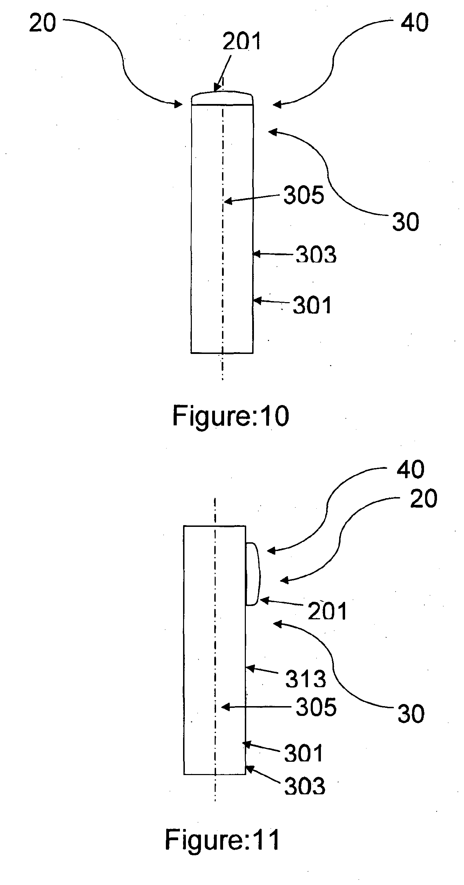

Referring now to Figure 10, there is shown a delivery device according to a third embodiment. The delivery device according to the third embodiment is similar in many respected to the first embodiment and corresponding reference numerals are used to identify similar parts. In this third embodiment, the handle 303 is generally straight and the first portion 200 is adapted to be releasably connected to the end thereof, whereby the delivery surface 201 defined by the first portion 20 is disposed transverse to the a longitudinal axis 305.

Referring now to Figure 11, there is shown a delivery device according to a fourth embodiment. The delivery device according to the fourth embodiment is similar in many respected to the first embodiment and corresponding reference numerals are used to identify similar parts. In this fourth embodiment, the first portion 20 is adapted to be realisably connected to a side 313 of the second portion 30. With this arrangement, the delivery surface 201 defined by the first portion 20 is generally aligned with the longitudinal extent of the handle 303. In other words, the delivery surface 201 protrudes sidewardly from the handle.

The present invention is set out in the appended claims. The embodiments, examples or aspects according to the present description which do not fall within the scope of said claims are provided for illustrative purposes only and do not form part of the present invention.

## Claims

1. A device (10) for delivering a cosmetic active agent to a dermal or mucosal surface comprising:
a) a first portion (20) configured to define a delivery surface (21) for presentation to a dermal or mucosal surface to deliver the cosmetic active agent (202); and
b) a second portion (30) configured as a body (301) to be held by a user further comprising a means for driving the passage of the cosmetic active agent into the dermal or mucosal surface
**characterised in that**: the first portion (20) and the second portion (30) are adapted to be releasably interconnected and the means (40) for driving the passage of the cosmetic active agent (202) into the dermal or mucosal surface is housed wholly in the second portion and comprises an electromagnetophoresis dermaportation device located immediately adjacent the point of interconnection (301) to the first portion.

2. The device (10) of claim 1 wherein the electromagnetophoresis dermaportation device housed in the second portion comprises:
an electromagnetic field generating device including a coil which has electrical connectivity at both ends to allow unidirectional flow of current;
a control device arranged to control said field generating device to alternately produce active and substantially inactive electromagnetic field portions, each said active electromagnetic field portion having a frequency of between 10Hz and 100Hz at a field strength of between 1 and 50 Gauss and comprising a plurality of generally rectangular electromagnetic field pulses wherein each electromagnetic field pulse has a duration of between 25µs and 100ms, each said substantially inactive electromagnetic field portion including no electromagnetic field pulses, wherein the duration of the inactive electromagnetic field portion is longer than the duration of the active electromagnetic field portion;
wherein during use when the electromagnetic field is incident on the dermal or mucosal surface, permeability is increased.

3. A device (10) according to claim 1 wherein the first portion (20) is configured as a cartridge adapted to hold the cosmetic active agent (202) and wherein the first portion (20) comprises a body (301) having an outer periphery defining an inner region adapted to accommodate the cosmetic active agent.

4. A device (10) according to claim 1 wherein the second portion (30) is configured as:
a) a handle (303) to be held by a user;
b) a glove to be worn by a user; or
c) a finger stall or finger sheath to be worn by a user;

5. A device (10) according to claim 1 wherein the first portion (20) is applied substantially or completely externally to the second portion (40).

6. A device (10) according to claim 1 wherein the electromagnetic field generating device housed in the second portion comprises a solid state switching device.

7. A device (10) according to claim 1 wherein the control device is arranged to produce an energisation signal useable to control switching of the solid state switching device to produce active and substantially inactive energisation signal portions, each active energisation signal portion including a plurality of energisation signal pulses and a substantially inactive energisation signal portion including no energisation signal pulses, and wherein the active energisation signal portion produces the active electromagnetic field portion and the inactive energisation signal portion produces the inactive electromagnetic field portion.

8. A device according (10) to claim 1 wherein the electromagnetophoresis dermaportation device produces and delivers an electromagnetic field to the dermal or mucosal surface wherein the field delivered is defined by a mnemonic profile of:
[(A-B-C-D)E], [(A₁-B₁-C₁-D₁)E₁]
where,
A and A₁ respectively define the number of 400µs time units that the electromagnetic field pulse is on for wherein each of A and A₁ is a number between 0.1 and 10;
B and B₁ respectively define is the number of 400µs time units the field is off for wherein B is a number between 0.1 and 100, while B₁ is a number between 0.1 and 100;
C is a number between 1 and 255, which defines the number of times the A and B combination is repeated, while C₁ is also a number between 1 and 255 and defines the number of times the A₁ and B₁ combination is repeated;
D and D₁ respectively define the number of 400µs time units that the field is off for wherein D is a number between 0 and 255, while D₁ is a number between 0 and 255;
E defines the number of times the A to D envelope is executed before moving onto the [(A₁-B₁-C₁-D₁)E₁] packet, while E₁ defines the number of times the A₁ to D₁ envelope is executed before moving onto the next mnemonic profile wherein E and E₁ are respectively numbers between 1 and 25; and
and wherein during use when the electromagnetic field is incident on the dermal or mucosal surface, the cellular environment in the surface is modified.

9. A device (10) according to claim 1 wherein the cosmetic active agent (202) is chosen from the list comprising: colouring agents including coloured cosmetics, concealing agents, skin lightening or whitening agents, tanning agents, pigments and dyes; sun protecting agents; cleaning agents including exfoliants and dermabrasives; anti-aging agents; moisturising agents, emollients, rehydrating agents and skin nourish agents; cleaning compounds; sloughing agents; pore treatment agents; oils, creams, gels and serums; antiperspirants, deodorants, perfumes and fragrances; anti-wrinkle agents, fillers, plumping agents and agents that alter the skin's topography; peptides; astringents; antioxidants; vitamins; nutrients; skin restorative agents.

10. A device according to claim 1 comprising a means to hold the cosmetic active agent (202) adapted for use as the first portion (20) and configured to define the delivery surface (201) for presentation to a dermal or mucosal surface to deliver the cosmetic active agent.

11. The device of claim 1 wherein the second portion (30) is, configured as:
a) a handle (303) to be held by a user;
b) a glove to be worn by a user; or
c) a finger stall or finger sheath to be worn by a user; and
wherein the second portion (30) further comprises the electromagnetophoresis dermaportation device for driving the passage of the cosmetic active agent (202) across the dermal or mucosal surface

12. A method for delivering a cosmetic active agent (202) to a dermal or mucosal surface comprising the step of:
moving a device (10) for delivering a cosmetic active agent to a dermal or mucosal surface over the dermal or mucosal surface
wherein the device for delivering a cosmetic active agent comprises
a first portion (20) configured to define a delivery surface (201) for presentation to a dermal or mucosal surface to deliver a cosmetic active agent (202); and
a second portion (30) configured as a body (301) to be held by a user **characterised in that**: the first portion (20) and the second portion (30) are adapted to be releasably interconnected, such that the cosmetic active agent (202) in the first portion is delivered to the dermal or mucosal surface; and the second portion (30) further comprises a means (40) for driving the passage of the cosmetic active agent into the dermal or mucosal surface, which means is housed wholly in the second portion, and is for driving the passage of the cosmetic active agent comprising an electromagnetophoresis dermaportation device located immediately adjacent the interconnection point of the first portion.

13. The method of claim 12 wherein the electromagnetophoresis dermaportation device comprises:
an electromagnetic field generating device including a coil which has electrical connectivity at both ends to allow unidirectional flow of current;
a control device arranged to control said field generating device to alternately produce active and substantially inactive electromagnetic field portions, each said active electromagnetic field portion having a frequency of between 10Hz and 100Hz at a field strength of between 1 and 50 Gauss and comprising a plurality of generally rectangular electromagnetic field pulses wherein each electromagnetic field pulse has a duration of between 25µs and 100ms, each said substantially inactive electromagnetic field portion including no electromagnetic field pulses, wherein the duration of the inactive electromagnetic field portion is longer than the duration of the active electromagnetic field portion;
wherein during use when the electromagnetic field is incident on the dermal or mucosal surface, permeability is increased.

## Patentansprüche

1. Vorrichtung (10) zum Verabreichen eines kosmetischen Wirkstoffs an eine dermale oder mukosale Oberfläche, umfassend:
a) einen ersten Abschnitt (20), der konfiguriert ist, um eine Verabreichungsfläche (21) zur Präsentation an eine dermale oder mukosale Oberfläche zu definieren, um den kosmetischen Wirkstoff (202) zu verabreichen; und
b) einen zweiten Abschnitt (30), der als ein Körper (301) konfiguriert ist, der von einem Benutzer gehalten werden soll, ferner umfassend ein Mittel zum Treiben des Durchgangs des kosmetischen Wirkstoffs in die dermale oder mukosale Oberfläche, **dadurch gekennzeichnet, dass**:
der erste Abschnitt (20) und der zweite Abschnitt (30) so ausgelegt sind, dass sie lösbar miteinander verbunden sind, und das Mittel (40) zum Treiben des Durchgangs des kosmetischen Wirkstoffs (202) in die dermale oder mukosale Oberfläche vollständig in dem zweiten Abschnitt untergebracht ist und eine Elektromagnetophorese-Dermaportationsvorrichtung umfasst, die unmittelbar neben dem Verbindungspunkt (301) zu dem ersten Abschnitt angeordnet ist.

2. Vorrichtung (10) nach Anspruch 1, wobei die in dem zweiten Abschnitt untergebrachte Elektromagnetophorese-Dermaportationsvorrichtung umfasst:
eine Vorrichtung zum Erzeugen eines elektromagnetischen Felds, beinhaltend eine Spule, die an beiden Enden eine elektrische Verbindung aufweist, um unidirektionalen Stromfluss zu ermöglichen;
eine Steuervorrichtung, die dazu eingerichtet ist, die Felderzeugungsvorrichtung zu steuern, um abwechselnd aktive und im Wesentlichen inaktive elektromagnetische Feldabschnitte zu erzeugen, wobei jeder aktive elektromagnetische Feldabschnitt eine Frequenz zwischen 10 Hz und 100 Hz bei einer Feldstärke zwischen 1 und 50 Gauß aufweist und eine Mehrzahl von im Allgemeinen rechteckigen elektromagnetischen Feldimpulsen umfasst, wobei jeder elektromagnetische Feldimpuls eine Dauer zwischen 25 µs und 100 ms hat, wobei jeder der im Wesentlichen inaktiven elektromagnetischen Feldabschnitt keine elektromagnetischen Feldimpulse beinhaltet, wobei die Dauer des inaktiven elektromagnetischen Feldabschnitts länger als die Dauer des aktiven elektromagnetischen Feldabschnitts ist;
wobei während der Verwendung, wenn das elektromagnetische Feld auf die dermale oder mukosale Oberfläche auftrifft, die Permeabilität erhöht wird.

3. Vorrichtung (10) nach Anspruch 1, wobei der erste Abschnitt (20) als eine Patrone ausgebildet ist, die dazu ausgelegt ist, den kosmetischen Wirkstoff (202) zu enthalten, und wobei der erste Abschnitt (20) einen Körper (301) mit einem Außenumfang umfasst, der einen inneren Bereich definiert, der so ausgelegt ist, dass er den kosmetischen Wirkstoff aufnimmt.

4. Vorrichtung (10) nach Anspruch 1, wobei der zweite Abschnitt (30) konfiguriert ist als:
a) ein Griff (303), der von einem Benutzer gehalten werden soll;
b) ein Handschuh, den von einem Benutzer getragen werden soll; oder
c) ein Fingerling oder eine Fingerhülle, der bzw. die von einem Benutzer getragen werden soll.

5. Vorrichtung (10) nach Anspruch 1, wobei der erste Abschnitt (20) im Wesentlichen oder vollständig außen auf dem zweiten Abschnitt (40) aufgebracht ist.

6. Vorrichtung (10) nach Anspruch 1, wobei die in dem zweiten Abschnitt untergebrachte Vorrichtung zum Erzeugen eines elektromagnetischen Felds eine Halbleiterschaltvorrichtung umfasst.

7. Vorrichtung (10) nach Anspruch 1 , wobei die Steuervorrichtung eingerichtet ist, ein Erregungssignal zu erzeugen, das verwendbar ist, um das Schalten der Halbleiterschaltvorrichtung steuern, um aktive und im Wesentlichen inaktive Erregungssignalabschnitte zu erzeugen, wobei jeder aktive Erregungssignalabschnitt eine Mehrzahl von Erregungssignalimpulsen beinhaltet und ein im Wesentlichen inaktiver Erregungssignalabschnitt keine Erregungssignalimpulse beinhaltet, und wobei der aktive Erregungssignalabschnitt den aktiven elektromagnetischen Feldabschnitt erzeugt und der inaktive Erregungssignalabschnitt den inaktiven elektromagnetischen Feldabschnitt erzeugt.

8. Vorrichtung (10) nach Anspruch 1, wobei die Elektromagnetophorese-Dermaportationsvorrichtung ein elektromagnetisches Feld erzeugt und an die dermale oder mukosale Oberfläche verabreicht, wobei das verabreichte Feld durch ein mnemonisches Profil wie folgt definiert ist:
[(A-B-C-D)E], [(A₁-B₁-C₁-D₁)E₁]
wobei A und A₁ jeweils die Anzahl von 400-µs-Zeiteinheiten definieren, für die der elektromagnetische Feldimpuls eingeschaltet ist, wobei A und A₁ jeweils eine Zahl zwischen 0,1 und 10 sind;
B und B₁ jeweils die Anzahl von 400-µs-Zeiteinheiten definieren, für die das Feld ausgeschaltet ist, wobei B eine Zahl zwischen 0,1 und 100 ist, während B₁ eine Zahl zwischen 0,1 und 100 ist;
C eine Zahl zwischen 1 und 255 ist, die definiert, wie oft die Kombination aus A und B wiederholt wird, während C₁ auch eine Zahl zwischen 1 und 255 ist und definiert, wie oft die Kombination aus A₁ und B₁ wiederholt wird;
D und D₁ jeweils die Anzahl von 400-µs-Zeiteinheiten definieren, für die das Feld ausgeschaltet ist, wobei D eine Zahl zwischen 0 und 255 ist, während D₁ eine Zahl zwischen 0 und 255 ist;
E definiert, wie oft die Hüllkurve von A bis D ausgeführt wird, bevor zum Paket [(A₁ - B₁-C₁-D₁)E₁] übergegangen wird, während E₁ definiert, wie oft die Hüllkurve von A₁ bis D₁ ausgeführt wird, bevor zum nächsten mnemonischen Profil übergegangen wird, wobei E und E₁ jeweils Zahlen zwischen 1 und 25 sind;
und wobei während der Verwendung, wenn das elektromagnetische Feld auf die dermale oder mukosale Oberfläche auftrifft, die zelluläre Umgebung in der Oberfläche modifiziert wird.

9. Vorrichtung (10) nach Anspruch 1, wobei der kosmetische Wirkstoff (202) aus der Liste ausgewählt ist, die umfasst:
Färbungsmittel, einschließlich farbiger Kosmetika, Abdeckungsmittel, Hautaufhellungsmittel oder Weißmacher, Bräunungsmittel, Pigmente und Farbstoffe;
Sonnenschutzmittel;
Reinigungsmittel, einschließlich Exfoliatonsmittel und Dermabrasionsmittel;
Anti-Aging-Mittel;
Feuchtigkeitsmittel, Weichmacher, Rehydratisierungsmittel und Hautpflegemittel; reinigende Verbindungen;
Verschorfungsmittel ;
Porenbehandlungsmittel;
Öle, Cremes, Gele und Seren;
Antitranspirantien, Deodorants, Parfums und Duftstoffe;
Antifaltenmittel, Füllstoffe, Straffungsmittel und Mittel, welche die Topographie der Haut verändern;
Peptide;
Adstringenzien;
Antioxidantien;
Vitamine;
Nährstoffe;
hautwiederherstellende Mittel.

10. Vorrichtung nach Anspruch 1, umfassend ein Mittel zum Enthalten des kosmetischen Wirkstoffs (202), das dazu ausgelegt ist, als der erste Abschnitt (20) verwendet zu werden, und das konfiguriert ist, um die Verabreichungsfläche (201) zur Präsentation an eine dermale oder mukosale Oberfläche zu definieren, um den kosmetischen Wirkstoff zu verabreichen.

11. Vorrichtung nach Anspruch 1, wobei der zweite Abschnitt (30) konfiguriert ist als:
a) ein Griff (303), der von einem Benutzer gehalten werden soll;
b) ein Handschuh, den von einem Benutzer getragen werden soll; oder
c) ein Fingerling oder eine Fingerhülle, der bzw. die von einem Benutzer getragen werden soll; und
wobei der zweite Abschnitt (30) ferner die Elektromagnetophorese-Dermaportationsvorrichtung zum Treiben des Durchgangs des kosmetischen Wirkstoffs (202) über die dermale oder mukosale Oberfläche umfasst.

12. Verfahren zum Verabreichen eines kosmetischen Wirkstoffs (202) an eine dermale oder mukosale Oberfläche, umfassend den folgenden Schritt:
Bewegen einer Vorrichtung (10) zum Verabreichen eines kosmetischen Wirkstoffs an eine dermale oder mukosale Oberfläche über der dermalen oder mukosalen Oberfläche, wobei die Vorrichtung zum Verabreichen eines kosmetischen Wirkstoffs umfasst: einen ersten Abschnitt (20), der konfiguriert ist, um eine Verabreichungsfläche (201) zur Präsentation an eine dermale oder mukosale Oberfläche zu definieren, um einen kosmetischen Wirkstoff (202) zu verabreichen; und
einen zweiten Abschnitt (30), der als ein Körper (301) konfiguriert ist, der von einem Benutzer gehalten werden soll, **dadurch gekennzeichnet ist, dass**:
der erste Abschnitt (20) und der zweite Abschnitt (30) so ausgelegt sind, dass sie lösbar miteinander verbunden sind, so dass der kosmetische Wirkstoff (202) in dem ersten Abschnitt an die dermale oder mukosale Oberfläche verabreicht wird; und
der zweite Abschnitt (30) ferner ein Mittel (40) zum Treiben des Durchgangs des kosmetischen Wirkstoffs in die dermale oder mukosale Oberfläche umfasst, wobei dieses vollständig in dem zweiten Abschnitt untergebracht ist, dem Treiben des Durchgangs des kosmetischen Wirkstoffs dient und eine Elektromagnetophorese-Dermaportationsvorrichtung umfasst, die unmittelbar neben dem Verbindungspunkt des ersten Abschnitts angeordnet ist.

13. Verfahren nach Anspruch 12, wobei die Elektromagnetophorese-Dermaportationsvorrichtung umfasst:
eine Vorrichtung zum Erzeugen eines elektromagnetischen Felds, beinhaltend eine Spule, die an beiden Enden eine elektrische Verbindung aufweist, um unidirektionalen Stromfluss zu ermöglichen;
eine Steuervorrichtung, die dazu eingerichtet ist, die Felderzeugungsvorrichtung zu steuern, um abwechselnd aktive und im Wesentlichen inaktive elektromagnetische Feldabschnitte zu erzeugen, wobei jeder aktive elektromagnetische Feldabschnitt eine Frequenz zwischen 10 Hz und 100 Hz bei einer Feldstärke zwischen 1 und 50 Gauß aufweist und eine Mehrzahl von im Allgemeinen rechteckigen elektromagnetischen Feldimpulsen umfasst, wobei jeder elektromagnetische Feldimpuls eine Dauer zwischen 25 µs und 100 ms hat, wobei jeder der im Wesentlichen inaktiven elektromagnetischen Feldabschnitt keine elektromagnetischen Feldimpulse beinhaltet, wobei die Dauer des inaktiven elektromagnetischen Feldabschnitts länger als die Dauer des aktiven elektromagnetischen Feldabschnitts ist;
wobei während der Verwendung, wenn das elektromagnetische Feld auf die dermale oder mukosale Oberfläche auftrifft, die Permeabilität erhöht wird.

## Revendications

1. Dispositif (10) permettant d'administrer un agent actif cosmétique à une surface dermique ou muqueuse, le dispositif comprenant :
a) une première partie (20) configurée pour définir une surface d'administration (21) pour une présentation à une surface dermique ou muqueuse afin d'administrer l'agent actif cosmétique (202) ; et
b) une seconde partie (30) configurée comme un corps (301) destiné à être tenu par un utilisateur, le dispositif comprenant en outre un moyen pour entraîner le passage de l'agent actif cosmétique dans la surface dermique ou muqueuse ;
**caractérisé en ce que** :
la première partie (20) et la seconde partie (30) sont conçues pour être reliées réciproquement de manière libérable, et **en ce que** le moyen (40) pour entraîner le passage de l'agent actif cosmétique (202) dans la surface dermique ou muqueuse est logé entièrement dans la seconde partie et comprend un dispositif de dermaportation par électromagnétophorèse situé immédiatement adjacent au point de liaison réciproque (301) avec la première partie.

2. Dispositif (10) selon la revendication 1, dans lequel le dispositif de dermaportation par électromagnétophorèse logé dans la seconde partie comprend :
un dispositif générateur de champ électromagnétique comprenant une bobine qui présente une connectivité électrique aux deux extrémités, de façon à permettre un flux unidirectionnel de courant ;
un dispositif de commande prévu pour amener ledit dispositif générateur de champ à produire alternativement des parties de champ électromagnétique actives et sensiblement inactives, chacune desdites parties de champ électromagnétique actives ayant une fréquence comprise entre 10 et 100 Hz à une intensité de champ comprise entre 1 et 50 Gauss et comprenant une pluralité d'impulsions de champ électromagnétique généralement rectangulaires, chaque impulsion de champ électromagnétique ayant une durée comprise entre 25 µs et 100 ms, chacune desdites parties de champ électromagnétique sensiblement inactives ne comprenant aucune impulsion de champ électromagnétique, la durée de la partie de champ électromagnétique inactive étant supérieure à la durée de la partie de champ électromagnétique active ;
la perméabilité étant augmentée pendant l'utilisation du dispositif, lorsque le champ électromagnétique est incident sur la surface dermique ou muqueuse.

3. Dispositif (10) selon la revendication 1, dans lequel la première partie (20) est configurée comme une cartouche conçue pour contenir l'agent actif cosmétique (202), et dans lequel la première partie (20) comprend un corps (301) ayant une périphérie extérieure définissant une région intérieure conçue pour recevoir l'agent actif cosmétique.

4. Dispositif (10) selon la revendication 1, dans lequel la seconde partie (30) est configurée comme :
a) une poignée (303) destinée à être tenue par un utilisateur ;
b) un gant destiné à être porté par un utilisateur ; ou
c) un doigtier ou une gaine de doigt destiné(e) à être porté(e) par un utilisateur.

5. Dispositif (10) selon la revendication 1, dans lequel la première partie (20) est appliquée sensiblement ou entièrement à l'extérieur de la seconde partie (40).

6. Dispositif (10) selon la revendication 1, dans lequel le dispositif générateur de champ électromagnétique logé dans la seconde partie comprend un dispositif de commutation à semi-conducteurs.

7. Dispositif (10) selon la revendication 1, dans lequel le dispositif de commande est prévu pour produire un signal d'excitation pouvant être utilisé pour commander la commutation du dispositif de commutation à semi-conducteurs afin de produire des parties de signal d'excitation actives et sensiblement inactives, chaque partie de signal d'excitation active comprenant une pluralité d'impulsions de signal d'excitation et une partie de signal d'excitation sensiblement inactive ne comprenant aucune impulsion de signal d'excitation, et dans lequel la partie de signal d'excitation active produit la partie de champ électromagnétique active et la partie de signal d'excitation inactive produit la partie de champ électromagnétique inactive.

8. Dispositif selon (10) à la revendication 1, dans lequel le dispositif de dermaportation par électromagnétophorèse produit et administre un champ électromagnétique à la surface dermique ou muqueuse, le champ administré étant défini par un profil mnémonique de :
[(A-B-C-D)E], [(A₁-B₁-C₁-D₁)E₁], où
A et A₁ définissent respectivement le nombre d'unités de temps de 400 µs pour lesquelles l'impulsion de champ électromagnétique est activée, chacun de A et A₁ étant un nombre compris entre 0,1 et 10 ;
B et B₁ définissent respectivement le nombre d'unités de temps de 400 µs pour lesquelles le champ est désactivé, B étant un nombre compris entre 0,1 et 100, tandis que B₁ est un nombre compris entre 0,1 et 100 ;
C est un nombre compris entre 1 et 255, lequel définit le nombre de récurrences de la combinaison A et B, tandis que C₁ est également un nombre compris entre 1 et 255 et définit le nombre de récurrences de la combinaison A₁ et B₁ ;
D et D₁ définissent respectivement le nombre d'unités de temps de 400 µs pour lesquelles le champ est désactivé, D étant un nombre compris entre 0 et 255, tandis que D₁ est un nombre compris entre 0 et 255 ;
E définit le nombre de fois où l'enveloppe A à D est exécutée avant de passer au paquet [(A₁-B₁-C₁-D₁)E₁], tandis que E₁ définit le nombre de fois où l'enveloppe A₁ à D₁ est exécutée avant de passer au profil mnémonique suivant, E et E₁ étant respectivement des nombres compris entre 1 et 25 ; et
l'environnement cellulaire dans la surface étant modifié pendant l'utilisation du dispositif, lorsque le champ électromagnétique est incident sur la surface dermique ou muqueuse.

9. Dispositif (10) selon la revendication 1, dans lequel l'agent actif cosmétique (202) est choisi dans la liste comprenant :
des agents colorants dont des agents cosmétiques colorés, des agents masquants, des agents éclaircissants ou blanchissants pour la peau, des agents tannants, des pigments et des colorants ;
des agents de protection solaire ;
des agents nettoyants dont des exfoliants et des dermabrasifs ;
des agents anti-âge ;
des agents hydratants, des émollients, des agents réhydratants et des agents nourrissants pour la peau ; des composés de nettoyage ;
des agents de desquamation ;
des agents de traitement des pores ;
des huiles, des crèmes, des gels et des sérums ;
des antisudorifiques, des déodorants, des parfums et des fragrances ;
des agents anti-rides, des charges, des agents repulpants et des agents qui altèrent la topographie de la peau ;
des peptides ;
des astringents ;
des antioxydants ;
des vitamines ;
des nutriments ;
des agents de restauration de la peau.

10. Dispositif selon la revendication 1 comprenant un moyen pour tenir l'agent actif cosmétique (202), conçu pour une utilisation comme première partie (20) et configuré pour définir la surface d'administration (201) pour une présentation à une surface dermique ou muqueuse afin d'administrer le principe actif cosmétique.

11. Dispositif selon la revendication 1, dans lequel la seconde partie (30) est configurée comme :
a) une poignée (303) destinée à être tenue par un utilisateur ;
b) un gant destiné à être porté par un utilisateur ; ou
c) un doigtier ou une gaine de doigt destiné(e) à être porté(e) par un utilisateur ; et
la seconde partie (30) comprenant en outre le dispositif de dermaportation par électromagnétophorèse pour entraîner le passage de l'agent actif cosmétique (202) à travers la surface dermique ou muqueuse.

12. Procédé permettant d'administrer un agent actif cosmétique (202) à une surface dermique ou muqueuse, comprenant l'étape :
de déplacement d'un dispositif (10) pour administrer un agent actif cosmétique à une surface dermique ou muqueuse sur la surface dermique ou muqueuse, le dispositif permettant d'administrer un agent actif cosmétique comprenant une première partie (20) configurée pour définir une surface d'administration (201) pour une présentation à une surface dermique ou muqueuse afin d'administrer un agent actif cosmétique (202) ; et
une seconde partie (30) configurée comme un corps (301) destiné à être tenu par un utilisateur, **caractérisé en ce que** :
la première partie (20) et la seconde partie (30) sont conçues pour être reliées réciproquement de manière libérable, de sorte que l'agent actif cosmétique (202) dans la première partie soit administré à la surface dermique ou muqueuse ; et
la seconde partie (30) comprend en outre un moyen (40) pour entraîner le passage de l'agent actif cosmétique dans la surface dermique ou muqueuse, lequel moyen est entièrement logé dans la seconde partie, et sert à entraîner le passage de l'agent actif cosmétique comprenant un dispositif de dermaportation par électromagnétophorèse situé immédiatement adjacent au point de liaison réciproque avec la première partie.

13. Procédé selon la revendication 12, dans lequel le dispositif de dermaportation par électromagnétophorèse comprend :
un dispositif générateur de champ électromagnétique comprenant une bobine qui présente une connectivité électrique aux deux extrémités, de façon à permettre un flux unidirectionnel de courant ;
un dispositif de commande prévu pour amener ledit dispositif générateur de champ à produire alternativement des parties de champ électromagnétique actives et sensiblement inactives, chacune desdites parties de champ électromagnétique actives ayant une fréquence comprise entre 10 et 100 Hz à une intensité de champ comprise entre 1 et 50 Gauss et comprenant une pluralité d'impulsions de champ électromagnétique généralement rectangulaires, chaque impulsion de champ électromagnétique ayant une durée comprise entre 25 µs et 100 ms, chacune desdites parties de champ électromagnétique sensiblement inactives ne comprenant aucune impulsion de champ électromagnétique, la durée de la partie de champ électromagnétique inactive étant supérieure à la durée de la partie de champ électromagnétique active ;
la perméabilité étant augmentée pendant l'utilisation du dispositif, lorsque le champ électromagnétique est incident sur la surface dermique ou muqueuse.
